(19) Europäisches Patentamt
European Patent Office
Office européen des brevets

(11) **EP 4 745 284 A1**

(12) **EUROPEAN PATENT APPLICATION**
published in accordance with Art. 153(4) EPC

(43) Date of publication:
**20.05.2026 Bulletin 2026/21**

(21) Application number: **23945597.5**

(22) Date of filing: **07.10.2023**

(51) International Patent Classification (IPC):
***D04H 1/724*** (2012.01)

(86) International application number:
**PCT/CN2023/123169**

(87) International publication number:
**WO 2025/015716 (23.01.2025 Gazette 2025/04)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(30) Priority: **14.07.2023 CN 202310863987**

(71) Applicant: **Kingwills Advanced Materials Co., Ltd.
Nantong, Jiangsu 226017 (CN)**

(72) Inventor: **YE, Kongmeng
Nantong, Jiangsu 226017 (CN)**

(74) Representative: **Balder IP Law, S.L.
Paseo de la Castellana 93
5ª planta
28046 Madrid (ES)**

(54) **FLASHSPUN MATERIAL HAVING LOW HAZE ATTENUATION RATE**

(57)    The present disclosure relates to a flash evaporation material with a low haze attenuation rate, wherein the raw material of the flash evaporation material comprises polyethylene, and a grammage of a flash spun non-woven fabric is greater than 35 g/m$^2$, and a compression specific power P of the flash evaporation material is greater than 0.07 gf·cm/cm$^2$; the compression specific power is tested by a KES style tester, and the compression specific power is tested by FB3; a haze attenuation rate $\triangle W$ of the flash evaporation material is 0.3%-1.5%; $\triangle W=(W_0-W_5)/W_0*100\%$; a rate attenuation rate $\triangle S$ of a heat shielding of the flash evaporation material is 7%-15%; $\triangle S=(S_0-S_5)/S_0*100\%$. The present application utilizes the modification of a spinning raw material to enhance the performance of the flash evaporation material and expand its application.

**EP 4 745 284 A1**

**Description**

**TECHNICAL FIELD**

The present disclosure belongs to the field of flash evaporation technology, and in particular relates to a flash evaporation material with a low haze attenuation rate.

**BACKGROUND**

Flash evaporation spinning involves dissolving PE, PP and other similar polymers in a certain spinning solvent to produce a spinning solution, which is then ejected from the spinneret. Due to the rapid evaporation of the solvent, the polymer is re-solidified into fibers, which are adsorbed onto a lapping curtain to directly form a web, and then are prepared into a flash evaporation material through hot pressing and winding processes. The current polymer raw material for flash evaporation method is PE material, and a polyethylene non-woven fabric produced by flash evaporation method has many excellent properties, such as excellent waterproof and breathable properties; the sheet has excellent strength, tear resistance, puncture resistance, and rupture resistance; the sheet generally does not fuzz or produce dust; and it has excellent performance over a wide temperature range, etc. Therefore, the polyethylene non-woven fabric produced by flash evaporation method is widely used in packaging materials, protective clothing, cover cloths, and printing substrates.

Chinese Patent Publication No. CN114687057A relates to a flash evaporation sheet and an application thereof, wherein raw materials of the flash evaporation sheet are polyethylene and a modifier; an attenuation value $\triangle C$ of a bending length of the flash evaporation sheet is 0.1-0.4 ; $\triangle C=1-C2/C1$; C1 is the bending length of the flash evaporation sheet that has not undergone aging treatment; C2 is the bending length of the flash evaporation sheet after aging treatment; and the bending length C2 of the flash evaporation sheet after aging treatment is 4 cm-10 cm. While maintaining brightness, the flash evaporation sheet of the present application also has a lower decrease in bending length, which can extend the service life of the product and expand the scope of application.

Chinese Patent Publication No. CN114687069A relates to a multifunctional polymer non-woven fabric and a fabric thereof, wherein an attenuation rate A E of an elongation at break is 0.20-0.50; A E = 1-E2/E1; E1 is the elongation at break of the multifunctional polymer non-woven fabric that has not undergone aging test treatment; E2 is the elongation at break of the multifunctional polymer non-woven fabric after aging test treatment; and the value of E2 is less than 0.5. The process of the present application is simple and widely used.

Chinese Patent Publication No. CN114687068A relates to an improved flash evaporation non-woven fabric. The present disclosure aims at the problem in the prior art that a brightness of the flash evaporation non-woven fabric will decrease after a period of use, and provides an improved flash evaporation non-woven fabric. A loss rate AF of a D65 fluorescence brightness of the improved flash evaporation non-woven fabric is 0.20-0.30; wherein ▲F=1-F2/F1; F1 is the D65 fluorescence brightness of the non-woven fabric that has not undergone aging treatment; F2 is the D65 fluorescence brightness of the non-woven fabric after aging treatment; and the process conditions for aging treatment are: an irradiance within the wavelength range of 300 nm-400 nm is 60 w/m2$\pm$2 w/m$^2$, a black mark temperature is 65°C$\pm$2°C, an air temperature in a test chamber is 38°C$\pm$3°C, a relative humidity is 50%$\pm$10%, and a drying time is 1440 hours. The flash evaporation non-woven fabric of the present disclosure can maintain a high brightness after a period of use.

Chinese Patent Publication No. CN114108112A relates to a polyethylene sheet, wherein an aging rate $\triangle S$ of a tensile strength is 0.10-0.30; $\triangle S = 1-S2/S1$; S1 is the tensile strength of the non-woven fabric that has not undergone aging treatment, unit kN/m; S2 is the tensile strength of the non-woven fabric after aging treatment, unit kN/m; and the process conditions for aging treatment are: an irradiance within a wavelength range of 300 nm-400 nm is 60 w/m2$\pm$2 w/m$^2$, a black mark temperature is 65°C+2°C, an air temperature in a test chamber is 38°C$\pm$3°C, a relative humidity is 50%$\pm$10%, and a drying time is 1440 hours. The product of the present disclosure still has a good tensile strength after aging, thereby extending a service life of the product.

Chinese Patent Publication No. CN115537959A relates to a composite material, wherein a raw material of the composite material contains polyethylene; a shrinkage rate R is 0.2-0.7; a standard heat shrinkage strength or is 0.5 N/mm$^2$-3.5 N/mm$^2$; and an anti-mold level is less than level 2. The present application has a good shrinkage performance and better antibacterial and anti-mold effects, so it has a wide range of applications.

Chinese Patent Publication No. CN109154138A relates to a composite material comprising a non-woven vapor permeable sheet, a composite laminate, which comprises at least one water vapor permeable non-woven sheet having first and second surfaces and a fluorinated polymer coating on the first surface of the sheet, wherein (i) the fluorinated polymer is coated in an amount such that a total fluorine content of the coated non-woven sheet is present in an amount from 0.05 gsm to no greater than 0.4 gsm, and (ii) the composite laminate exhibits at least 60% retained hydrohead after exposure to wet wood.

U.S. Patent Publication No. US20160138197A1 relates to a flash spun plexifilamentary protofilament and a flash spun plexifilamentary fiber bundle with a BET surface area of less than 12 m$^2$/g and a crushing value of at least 0.9 mm/g, wherein the fiber bundle mainly comprises fibers formed from polyethylene, the fibers have a summary crystallinity index of less than 55%, and the fiber bundle has a sheet made of an elongation at break greater than 55%.

U.S. Patent Publication No. US8048513 flash spun sheet relates to an improved plexifilamentary sheet for use in a protective clothing and a filter media, wherein the material is composed of substantially continuous polyethylene plexifilamentary fiber bundles and has a Frazier permeability, normalized to 1.0 oz/yd$^2$, of at least 2 cfm/ft$^2$.

In the prior art, there are few studies on a heat shielding performance and a haze performance of the flash evaporation material, and there is a problem that the heat shielding performance and the haze performance of the flash evaporation material decrease after a period of use.

## SUMMARY OF THE INVENTION

The purpose of the present disclosure is to provide a flash evaporation material with a low haze attenuation rate to solve the above-mentioned problems.

In order to achieve the above-mentioned objects, the present disclosure adopts the following technical solutions:

a flash evaporation material with a low haze attenuation rate, wherein a raw material of the flash evaporation material comprises polyethylene, and a grammage of a flash spun non-woven fabric is greater than 35 g/m$^2$,
a compression specific power P of the flash evaporation material is greater than 0.07 gf·cm/cm$^2$;
the compression specific power is tested by a KES style tester, and the compression specific power is tested by FB3;
a haze attenuation rate $\triangle$W of the flash evaporation material is 0.3%-1.5%;

$$\triangle W = (W_0 - W_5)/W_0 * 100\%;$$

an attenuation rate $\triangle$S of a heat shielding rate of the flash evaporation material is 7%-15%;

$$\triangle S = (S_0 - S_5)/S_0 * 100\%$$

tests for a heat shielding rate, a haze and a transverse tensile strength:

(1) placing a sample at 25°C and a relative humidity of 65% for 24 hours; then measuring and recording a shading index thereof as an initial heat shielding rate $S_0$, measuring and recording a haze thereof as an initial haze $W_0$, and measuring and recording a transverse tensile strength as a transverse tensile strength $CTS_0$;
(2) then exposing the sample to a dry and hot atmosphere of 90°C for 6 hours, and then cooling at 25°C and a relative humidity of 65% for 24 hours;
(3) repeating the operation of step (2), and after treatment for 4 times, measuring and recording a shading index thereof as a final heat shielding rate $S_5$, measuring and recording a haze thereof as a final haze $W_5$, and measuring and recording a transverse tensile strength thereof as a final transverse tensile strength $CTS_5$;

the haze is tested according to the national standard GB/T2410-2008; and
the heat shielding rate is measured according to the national standard GB/T41560-2022.

Tensile strength is determined according to the national standard GB/T 12914-2018. Tensile strength is a maximum tension that a unit width specimen can withstand before fracture under specified test conditions.

The grammage of the flash spun non-woven fabric is less than 75 g/m$^2$.

The grammage of the flash spun non-woven fabric is less than 65 g/m$^2$.

The compression specific work P of the flash spun non-woven fabric is greater than 0.15 gf·cm/cm$^2$.

The compression specific work P of the flash spun non-woven fabric is less than 2 gf·cm/cm$^2$.

The compression specific work P of the flash spun non-woven fabric is less than 1 gf·cm/cm$^2$.

The haze attenuation rate $\triangle$W of the flash evaporation material is 0.6%-0.9%.

The haze attenuation rate $\triangle$W of the flash evaporation material is 0.9%-1.3%.

The attenuation rate of the heat shielding rate of the flash evaporation material is 7%-10%.

The attenuation rate of the heat shielding rate of the flash evaporation material is 10%-14%.

The attenuation rate $\triangle$CTS of the transverse tensile strength of the flash evaporation material is 2%-10%;

$$\triangle CTS = (CTS_0 - CTS_5)/CTS_0 * 100\%;$$

and

the transverse tensile strength $CTS_0$ is greater than 1.4 KN/m.

The attenuation rate $\triangle CTS$ of the transverse tensile strength of the flash evaporation material is 3%-5%.

The attenuation rate $\triangle CTS$ of the transverse tensile strength of the flash evaporation material is 5%-7%.

The attenuation rate $\triangle CTS$ of the transverse tensile strength of the flash evaporation material is 7%-9%.

A preparation method of a flash evaporation material with a low haze attenuation rate, which mainly includes the preparation of a spinning solution and the preparation of a flash evaporation material;

wherein,

preparation of a spinning solution: dissolving a spinning raw material in a spinning solvent to obtain a spinning solution; the spinning raw material includes polyethylene and a porous carbon material loaded with zinc oxide;

a mass fraction of the porous carbon material loaded with zinc oxide in the spinning raw material is 3%-6%;

a preparation method of the porous carbon material loaded with zinc oxide, which includes specific technical steps:

(1) preparation of a solution of silica nanoparticles surface-coated with polyvinyl alcohol:

adding silica nanoparticles to a deionized water for ultrasonic dispersion to obtain a dispersion, then adding a polyvinyl alcohol powder to the dispersion while heating and stirring, controlling a heating temperature to 80°C-100°C for dissolution and dispersion, waiting for the polyethylene to be dissolved and dispersed, and performing a reaction for 36-60 hours after the polyvinyl alcohol solution is dissolved, and then removing the undissolved polyvinyl alcohol by filtration to obtain a coating solution, which is a solution of silica nanoparticles surface-coated with polyvinyl alcohol;

a mass ratio of the silica nanoparticles to the polyvinyl alcohol powder is 1:9-1:12;

(2) preparation of a zinc-containing porous carbon precursor dispersion

further adding the solution of silica nanoparticles surface-coated with polyvinyl alcohol prepared in step (1) to a zinc nitrate aqueous solution to continue a first-order reaction, thus making zinc and polyvinyl alcohol perform a chelation reaction and being loaded on the surface of the silica nanoparticles, then using an alkali to slowly adjust a pH value of a mixed solution to 11-12, and continuing a second-order reaction under alkali conditions to obtain the zinc-containing porous carbon precursor dispersion;

a first-order reaction time is 30-48 hours, and a reaction temperature is 60°C-80°C;

the alkali can specifically be 0.1 mol/L sodium hydroxide solution or potassium hydroxide solution;

a second-order reaction is performed under alkaline conditions, and a reaction time is 4-8 hours;

in this step, an internal silicon dioxide is etched, and at the same time, internal zinc ions generate a zinc hydroxide precipitation in an alkaline solution, achieving simultaneous silicon dioxide etching and zinc hydroxide precipitation;

(3) preparation of a porous carbon material loaded with zinc oxide:

finally, separating the zinc-containing porous carbon precursor dispersion in step (2) by centrifugation, and taking a sediment from a lower layer to obtain a zinc-containing porous carbon precursor; performing aerobic calcination on the zinc-containing porous carbon precursor in the lower layer aerobically at 180°C-200°C for 15-30 minutes, and then rapidly heating up to 440°C-450°C within 1-5 minutes, performing anaerobic calcination for 45-60 minutes, and obtaining the porous carbon material loaded with zinc oxide after natural cooling.

During the calcination process, performing aerobic calcination first, due to the aerobic pre-oxidation carbonization of polyvinyl alcohol structure at 180°C, and the oxidation decomposition of zinc hydroxide to produce zinc oxide, and then transferring to high-temperature anaerobic carbonization, and making a carbon material formed in the anaerobic environment perform oxidation-reduction reaction with zinc oxide, ultimately producing an excellent zinc oxide, avoiding the dispersion problem of conventional blending and adding, and making zinc oxide generate on the surface of the carbon material to replace a reduced carbon material, forming a relatively stable porous carbon structure doped with zinc oxide. The porous carbon structure doped with zinc oxide is filled in gaps between flash evaporation fibers of the flash evaporation material, reducing the transmission of light and increasing the emission and refraction of light. Zinc oxide has a good emission performance, thereby improving a heat shielding performance of the product.

The spinning solvent includes a surfactant and a solvent; and

the surfactant in the spinning solvent is 500 ppm-2000 ppm.

The surfactant is polyvinylpyrrolidone.

The solvents include aromatic hydrocarbons, aliphatic hydrocarbons, cycloaliphatic hydrocarbons, unsaturated hydrocarbons, halogenated hydrocarbons, and their analogues.

Preparation of a flash evaporation material:

making the prepared spinning solution enter into a spinning chamber for flash evaporation spinning, and obtaining a flash evaporation fiber through a nozzle of a spinning component, then performing laying and hot pressing processes to the flash evaporation fiber to obtain a flash evaporation material.

a temperature of the flash evaporation spinning is 200°C-220°C; and

a temperature of the hot pressing is 105°C-115°C.

Compared with the prior art, the present disclosure has the following positive effects:
the present disclosure has found through experiments that the haze attenuation rate and the heat shielding rate attenuation rate of the flash evaporation material are at least closely related to factors such as the content of the porous carbon material loaded with zinc oxide in the spinning raw material and the content of the surfactant in the spinning solvent, and provides a novel preparation process for the flash evaporation material, which enables the flash evaporation material to have a lower haze attenuation rate and a lower attenuation rate of the heat shielding rate, thus overcoming the problem of significant decline in the heat shielding performance and the haze performance of the flash evaporation material after a period of use in the prior art.

## DETAILED DESCRIPTION OF THE INVENTION

The present disclosure will be described in further detail below in conjunction with specific embodiments.

Example 1

This embodiment provides a flash evaporation material with a low haze attenuation rate, which mainly includes the preparation of a spinning solution and the preparation of a flash evaporation material;
wherein,

preparation of a spinning solution: dissolving a spinning raw material in a spinning solvent to obtain a spinning solution;
the spinning raw material includes polyethylene and a porous carbon material loaded with zinc oxide;
a mass fraction of the porous carbon material loaded with zinc oxide in the spinning raw material is 3%;
a preparation method of the porous carbon material loaded with zinc oxide, which includes specific technical steps:

(1) preparation of a solution of silica nanoparticles surface-coated with polyvinyl alcohol:

adding silica nanoparticles to a deionized water for ultrasonic dispersion to obtain a dispersion, then adding a polyvinyl alcohol powder to the dispersion while heating and stirring, controlling a heating temperature to 80°C-100°C for dissolution and dispersion, waiting for the polyethylene to be dissolved and dispersed, and performing a reaction for 36-60 hours after the polyvinyl alcohol solution is dissolved, and then removing the undissolved polyvinyl alcohol by filtration to obtain a coating solution, which is a solution of silica nanoparticles surface-coated with polyvinyl alcohol;
a mass ratio of the silica nanoparticles to the polyvinyl alcohol powder is 1:9;

(2) preparation of a zinc-containing porous carbon precursor dispersion

further adding the solution of silica nanoparticles surface-coated with polyvinyl alcohol prepared in step (1) to a zinc nitrate aqueous solution to continue a first-order reaction, thus making zinc and polyvinyl alcohol perform a chelation reaction and being loaded on the surface of the silica nanoparticles, then using an alkali to slowly adjust a pH value of a mixed solution to 11-12, and continuing a second-order reaction under alkali conditions to obtain the zinc-containing porous carbon precursor dispersion;
a first-order reaction time is 30 hours, and a reaction temperature is 60°C-80°C;
the alkali can specifically be 0.1 mol/L sodium hydroxide solution or potassium hydroxide solution;
a second-order reaction is performed under alkaline conditions, and a reaction time is 5 hours;
in this step, an internal silicon dioxide is etched, and at the same time, internal zinc ions generate a zinc hydroxide precipitation in an alkaline solution, achieving simultaneous silicon dioxide etching and zinc hydroxide precipitation;

(3) preparation of a porous carbon material loaded with zinc oxide:

finally, separating the zinc-containing porous carbon precursor dispersion in step (2) by centrifugation, and taking a sediment from a lower layer to obtain a zinc-containing porous carbon precursor; performing aerobic calcination on the zinc-containing porous carbon precursor in the lower layer aerobically at 180°C-200°C for 15-30 minutes, and then rapidly heating up to 440°C-450°C within 1-5 minutes, performing anaerobic calcination for 45-60 minutes, and obtaining the porous carbon material loaded with zinc oxide after natural cooling.

The spinning solvent includes a surfactant and a solvent; and
the surfactant in the spinning solvent is 700 ppm.

The surfactant is polyvinylpyrrolidone.

The solvent is chloroethane, dichloromethane, 1,1-dichloro-2,2-difluoroethane, and 1,1,2,2,3,3,4,4-octafluorobutane, with a mass ratio of 4:4:1:1.

Preparation of a flash evaporation material:
making the prepared spinning solution enter into a spinning chamber for flash evaporation spinning, and obtaining a flash evaporation fiber through a nozzle of a spinning component, then performing laying and hot pressing processes to the flash evaporation fiber to obtain a flash evaporation material.

A temperature of the flash evaporation spinning is 200°C; and
a temperature of the hot pressing is 105°C.

A performance test data of the flash evaporation material prepared in this embodiment is shown in Table 1.

Example 2

This embodiment provides a flash evaporation material with a low haze attenuation rate, which mainly includes the preparation of a spinning solution and the preparation of a flash evaporation material;
wherein,

preparation of a spinning solution: dissolving a spinning raw material in a spinning solvent to obtain a spinning solution;
the spinning raw material includes polyethylene and a porous carbon material loaded with zinc oxide;
a mass fraction of the porous carbon material loaded with zinc oxide in the spinning raw material is 4.5%;
a preparation method of the porous carbon material loaded with zinc oxide, which includes specific technical steps:

(1) preparation of a solution of silica nanoparticles surface-coated with polyvinyl alcohol:

adding silica nanoparticles to a deionized water for ultrasonic dispersion to obtain a dispersion, then adding a polyvinyl alcohol powder to the dispersion while heating and stirring, controlling a heating temperature to 80°C-100°C for dissolution and dispersion, waiting for the polyethylene to be dissolved and dispersed, and performing a reaction for 36-60 hours after the polyvinyl alcohol solution is dissolved, and then removing the undissolved polyvinyl alcohol by filtration to obtain a coating solution, which is a solution of silica nanoparticles surface-coated with polyvinyl alcohol;
a mass ratio of the silica nanoparticles to polyvinyl alcohol powder is 1:10.5;

(2) preparation of a zinc-containing porous carbon precursor dispersion

further adding the solution of silica nanoparticles surface-coated with polyvinyl alcohol prepared in step (1) to a zinc nitrate aqueous solution to continue a first-order reaction, thus making zinc and polyvinyl alcohol perform a chelation reaction and being loaded on the surface of the silica nanoparticles, then using an alkali to slowly adjust a pH value of a mixed solution to 11-12, and continuing a second-order reaction under alkali conditions to obtain the zinc-containing porous carbon precursor dispersion;
a first-order reaction time is 40 hours, and a reaction temperature is 60°C-80°C;
the alkali can specifically be 0.1 mol/L sodium hydroxide solution or potassium hydroxide solution;
a second-order reaction is performed under alkaline conditions, and a reaction time is 6 hours;
in this step, an internal silicon dioxide is etched, and at the same time, internal zinc ions generate a zinc hydroxide precipitation in an alkaline solution, achieving simultaneous silicon dioxide etching and zinc hydroxide precipitation;

(3) preparation of a porous carbon material loaded with zinc oxide:
finally, separating the zinc-containing porous carbon precursor dispersion in step (2) by centrifugation, and taking a sediment from a lower layer to obtain a zinc-containing porous carbon precursor; performing aerobic calcination on the zinc-containing porous carbon precursor in the lower layer aerobically at 180°C-200°C for 15-30 minutes, and then rapidly heating up to 440°C-450°C within 1-5 minutes, performing anaerobic calcination for 45-60 minutes, and obtaining the porous carbon material loaded with zinc oxide after natural cooling.

The spinning solvent includes a surfactant and a solvent; and the surfactant in the spinning solvent is 1200 ppm.

The surfactant is polyvinylpyrrolidone.

The solvent is chloroethane, dichloromethane, 1,1-dichloro-2,2-difluoroethane, and 1,1,2,2,3,3,4,4-octafluorobutane, with a mass ratio of 4:4:1:1.

Preparation of a flash evaporation material:

making the prepared spinning solution enter into a spinning chamber for flash evaporation spinning, and obtaining a flash evaporation fiber through a nozzle of a spinning component, then performing laying and hot pressing processes to the flash evaporation fiber to obtain a flash evaporation material.

A temperature of the flash evaporation spinning is 210°C; and a temperature of the hot pressing is 110°C.

A performance test data of the flash evaporation material prepared in this embodiment is shown in Table 1.

Example 3

This embodiment provides a flash evaporation material with a low haze attenuation rate, which mainly includes the preparation of a spinning solution and the preparation of a flash evaporation material;
wherein,

preparation of a spinning solution: dissolving a spinning raw material in a spinning solvent to obtain a spinning solution;
the spinning raw material includes polyethylene and a porous carbon material loaded with zinc oxide;
a mass fraction of the porous carbon material loaded with zinc oxide in the spinning raw material is 6%;
a preparation method of the porous carbon material loaded with zinc oxide, which includes specific technical steps:

(1) preparation of a solution of silica nanoparticles surface-coated with polyvinyl alcohol:

adding silica nanoparticles to a deionized water for ultrasonic dispersion to obtain a dispersion, then adding a polyvinyl alcohol powder to the dispersion while heating and stirring, controlling a heating temperature to 80°C-100°C for dissolution and dispersion, waiting for the polyethylene to be dissolved and dispersed, and performing a reaction for 36-60 hours after the polyvinyl alcohol solution is dissolved, and then removing the undissolved polyvinyl alcohol by filtration to obtain a coating solution, which is a solution of silica nanoparticles surface-coated with polyvinyl alcohol;
a mass ratio of the silica nanoparticles to polyvinyl alcohol powder is 1:12;

(2) preparation of a zinc-containing porous carbon precursor dispersion

further adding the solution of silica nanoparticles surface-coated with polyvinyl alcohol prepared in step (1) to a zinc nitrate aqueous solution to continue a first-order reaction, thus making zinc and polyvinyl alcohol perform a chelation reaction and being loaded on the surface of the silica nanoparticles, then using an alkali to slowly adjust a pH value of a mixed solution to 11-12, and continuing a second-order reaction under alkali conditions to obtain the zinc-containing porous carbon precursor dispersion;
a first-order reaction time is 48 hours, and a reaction temperature is 60°C-80°C;
the alkali can specifically be 0.1 mol/L sodium hydroxide solution or potassium hydroxide solution;
a second-order reaction is performed under alkaline conditions, and a reaction time is 8 hours;
in this step, an internal silicon dioxide is etched, and at the same time, internal zinc ions generate a zinc hydroxide precipitation in an alkaline solution, achieving simultaneous silicon dioxide etching and zinc hydroxide precipitation;

(3) preparation of a porous carbon material loaded with zinc oxide:
finally, separating the zinc-containing porous carbon precursor dispersion in step (2) by centrifugation, and taking a sediment from a lower layer to obtain a zinc-containing porous carbon precursor; performing aerobic calcination on the zinc-containing porous carbon precursor in the lower layer aerobically at 180°C-200°C for 15-30 minutes, and then rapidly heating up to 440°C-450°C within 1-5 minutes, performing anaerobic calcination for 45-60 minutes, and obtaining the porous carbon material loaded with zinc oxide after natural cooling.

The spinning solvent includes a surfactant and a solvent; and the surfactant in the spinning solvent is 2000 ppm.

The surfactant is polyvinylpyrrolidone.

The solvent is chloroethane, dichloromethane, 1,1-dichloro-2,2-difluoroethane, and 1,1,2,2,3,3,4,4-octafluorobutane, with a mass ratio of 4:4:1:1.

Preparation of a flash evaporation material:

making the prepared spinning solution enter into a spinning chamber for flash evaporation spinning, and obtaining a flash evaporation fiber through a nozzle of a spinning component, then performing laying and hot pressing processes to the flash evaporation fiber to obtain a flash evaporation material.

A temperature of the flash evaporation spinning is 220°C; and a temperature of the hot pressing is 115°C.

A performance test data of the flash evaporation material prepared in this embodiment is shown in Table 1.

Comparative Example 1

This comparative example provides a flash evaporation material, which mainly includes the preparation of a spinning solution and the preparation of a flash evaporation material; wherein,

preparation of a spinning solution: dissolving a spinning raw material in a spinning solvent to obtain a spinning solution; the spinning raw material is polyethylene; the spinning solvent includes a surfactant and a solvent; and the surfactant in the spinning solvent is 1200 ppm.

The surfactant is polyvinylpyrrolidone.

The solvent is chloroethane, dichloromethane, 1,1-dichloro-2,2-difluoroethane, and 1,1,2,2,3,3,4,4-octafluorobutane, with a mass ratio of 4:4:1:1.

Preparation of a flash evaporation material:

making the prepared spinning solution enter into a spinning chamber for flash evaporation spinning, and obtaining a flash evaporation fiber through a nozzle of a spinning component, then performing laying and hot pressing processes to the flash evaporation fiber to obtain a flash evaporation material.

A temperature of the flash evaporation spinning is 210°C; and a temperature of the hot pressing is 110°C.

A performance test data of the flash evaporation material prepared in this comparative example is shown in Table 1.

Comparative Example 2

This comparative example provides a flash evaporation material, which mainly includes the preparation of a spinning solution and the preparation of a flash evaporation material; wherein,

preparation of a spinning solution: dissolving a spinning raw material in a spinning solvent to obtain a spinning solution; the spinning raw material includes polyethylene and a porous carbon material loaded with zinc oxide; a mass fraction of the porous carbon material loaded with zinc oxide in the spinning raw material is 1%; a preparation method of the porous carbon material loaded with zinc oxide is the same as in Example 2.

The spinning solvent includes a surfactant and a solvent; and the surfactant in the spinning solvent is 1200 ppm.

The surfactant is polyvinylpyrrolidone.

The solvent is chloroethane, dichloromethane, 1,1-dichloro-2,2-difluoroethane, and 1,1,2,2,3,3,4,4-octafluorobutane, with a mass ratio of 4:4:1:1.

Preparation of a flash evaporation material:

making the prepared spinning solution enter into a spinning chamber for flash evaporation spinning, and obtaining a flash evaporation fiber through a nozzle of a spinning component, then performing laying and hot pressing processes to the flash evaporation fiber to obtain a flash evaporation material.

A temperature of the flash evaporation spinning is 210°C; and a temperature of the hot pressing is 110°C.

**EP 4 745 284 A1**

A performance test data of the flash evaporation material prepared in this comparative example is shown in Table 1.

Comparative Example 3

This comparative example provides a flash evaporation material, which mainly includes the preparation of a spinning solution and the preparation of a flash evaporation material; wherein,

preparation of a spinning solution: dissolving a spinning raw material in a spinning solvent to obtain a spinning solution; the spinning raw material includes polyethylene and a porous carbon material loaded with zinc oxide; a mass fraction of the porous carbon material loaded with zinc oxide in the spinning raw material is 2%; a preparation method of the porous carbon material loaded with zinc oxide is the same as in Example 2.

The spinning solvent includes a surfactant and a solvent; and the surfactant in the spinning solvent is 1200 ppm.
The surfactant is polyvinylpyrrolidone.
The solvent is chloroethane, dichloromethane, 1,1-dichloro-2,2-difluoroethane, and 1,1,2,2,3,3,4,4-octafluorobutane, with a mass ratio of 4:4:1:1.
Preparation of a flash evaporation material:
making the prepared spinning solution enter into a spinning chamber for flash evaporation spinning, and obtaining a flash evaporation fiber through a nozzle of a spinning component, then performing laying and hot pressing processes to the flash evaporation fiber to obtain a flash evaporation material.
A temperature of the flash evaporation spinning is 210°C; and
a temperature of the hot pressing is 110°C.
A performance test data of the flash evaporation material prepared in this comparative example is shown in Table 1.

Comparative Example 4

This comparative example provides a flash evaporation material, which mainly includes the preparation of a spinning solution and the preparation of a flash evaporation material; wherein,

preparation of a spinning solution: dissolving a spinning raw material in a spinning solvent to obtain a spinning solution; the spinning raw material includes polyethylene and a porous carbon material loaded with zinc oxide; a mass fraction of the porous carbon material loaded with zinc oxide in the spinning raw material is 8%; a preparation method of the porous carbon material loaded with zinc oxide is the same as in Example 2.

The spinning solvent includes a surfactant and a solvent; and the surfactant in the spinning solvent is 1200 ppm.
The surfactant is polyvinylpyrrolidone.
The solvent is chloroethane, dichloromethane, 1,1-dichloro-2,2-difluoroethane, and 1,1,2,2,3,3,4,4-octafluorobutane, with a mass ratio of 4:4:1:1.
Preparation of a flash evaporation material:
making the prepared spinning solution enter into a spinning chamber for flash evaporation spinning, and obtaining a flash evaporation fiber through a nozzle of a spinning component, then performing laying and hot pressing processes to the flash evaporation fiber to obtain a flash evaporation material.
A temperature of the flash evaporation spinning is 210°C; and
a temperature of the hot pressing is 110°C.
A performance test data of the flash evaporation material prepared in this comparative example is shown in Table 1.

Comparative Example 5

This comparative example provides a flash evaporation material, which mainly includes the preparation of a spinning solution and the preparation of a flash evaporation material; wherein,

preparation of a spinning solution: dissolving a spinning raw material in a spinning solvent to obtain a spinning solution;

the spinning raw material includes polyethylene and a porous carbon material loaded with zinc oxide;

a mass fraction of the porous carbon material loaded with zinc oxide in the spinning raw material is 9%;

a preparation method of the porous carbon material loaded with zinc oxide is the same as in Example 2.

The spinning solvent includes a surfactant and a solvent; and

the surfactant in the spinning solvent is 1200 ppm.

The surfactant is polyvinylpyrrolidone.

The solvent is chloroethane, dichloromethane, 1,1-dichloro-2,2-difluoroethane, and 1,1,2,2,3,3,4,4-octafluorobutane, with a mass ratio of 4:4:1:1.

Preparation of a flash evaporation material:

making the prepared spinning solution enter into a spinning chamber for flash evaporation spinning, and obtaining a flash evaporation fiber through a nozzle of a spinning component, then performing laying and hot pressing processes to the flash evaporation fiber to obtain a flash evaporation material.

A temperature of the flash evaporation spinning is 210°C; and

a temperature of the hot pressing is 110°C.

A performance test data of the flash evaporation material prepared in this comparative example is shown in Table 1.

Comparative Example 6

This comparative example provides a flash evaporation material, which mainly includes the preparation of a spinning solution and the preparation of a flash evaporation material;

wherein,

preparation of a spinning solution: dissolving a spinning raw material in a spinning solvent to obtain a spinning solution;

the spinning raw material includes polyethylene and a porous carbon material loaded with zinc oxide;

a mass fraction of the porous carbon material loaded with zinc oxide in the spinning raw material is 4.5%;

a preparation method of the porous carbon material loaded with zinc oxide is the same as in Example 2.

The spinning solvent is a solvent; and

the solvent is chloroethane, dichloromethane, 1,1-dichloro-2,2-difluoroethane, and 1,1,2,2,3,3,4,4-octafluorobutane, with a mass ratio of 4:4:1:1.

Preparation of a flash evaporation material:

making the prepared spinning solution enter into a spinning chamber for flash evaporation spinning, and obtaining a flash evaporation fiber through a nozzle of a spinning component, then performing laying and hot pressing processes to the flash evaporation fiber to obtain a flash evaporation material.

A temperature of the flash evaporation spinning is 210°C; and

a temperature of the hot pressing is 110°C.

A performance test data of the flash evaporation material prepared in this comparative example is shown in Table 1.

Comparative Example 7

This comparative example provides a flash evaporation material, which mainly includes the preparation of a spinning solution and the preparation of a flash evaporation material;

wherein,

preparation of a spinning solution: dissolving a spinning raw material in a spinning solvent to obtain a spinning solution;

the spinning raw material includes polyethylene and a porous carbon material loaded with zinc oxide;

a mass fraction of the porous carbon material loaded with zinc oxide in the spinning raw material is 4.5%;

a preparation method of the porous carbon material loaded with zinc oxide is the same as in Example 2.

The spinning solvent includes a surfactant and a solvent; and

the surfactant in the spinning solvent is 200 ppm.

The surfactant is polyvinylpyrrolidone.

The solvent is chloroethane, dichloromethane, 1,1-dichloro-2,2-difluoroethane, and 1,1,2,2,3,3,4,4-octafluorobutane, with a mass ratio of 4:4:1:1.

Preparation of a flash evaporation material:

making the prepared spinning solution enter into a spinning chamber for flash evaporation spinning, and obtaining a flash evaporation fiber through a nozzle of a spinning component, then performing laying and hot pressing processes to the flash evaporation fiber to obtain a flash evaporation material.

A temperature of the flash evaporation spinning is 210°C; and

a temperature of the hot pressing is 110°C.

A performance test data of the flash evaporation material prepared in this comparative example is shown in Table 1.

Comparative Example 8

This comparative example provides a flash evaporation material, which mainly includes the preparation of a spinning solution and the preparation of a flash evaporation material;
wherein,

preparation of a spinning solution: dissolving a spinning raw material in a spinning solvent to obtain a spinning solution;
the spinning raw material includes polyethylene and a porous carbon material loaded with zinc oxide;
a mass fraction of the porous carbon material loaded with zinc oxide in the spinning raw material is 4.5%;
a preparation method of the porous carbon material loaded with zinc oxide is the same as in Example 2.

The spinning solvent includes a surfactant and a solvent; and

the surfactant in the spinning solvent is 2000 ppm.

The surfactant is polyvinylpyrrolidone.

The solvent is chloroethane, dichloromethane, 1,1-dichloro-2,2-difluoroethane, and 1,1,2,2,3,3,4,4-octafluorobutane, with a mass ratio of 4:4:1:1.

Preparation of a flash evaporation material:

making the prepared spinning solution enter into a spinning chamber for flash evaporation spinning, and obtaining a flash evaporation fiber through a nozzle of a spinning component, then performing laying and hot pressing processes to the flash evaporation fiber to obtain a flash evaporation material.

A temperature of the flash evaporation spinning is 210°C; and

a temperature of the hot pressing is 110°C.

A performance test data of the flash evaporation material prepared in this comparative example is shown in Table 1.

Table 1 Performance test data of the flash evaporation material

|  | P gf·cm/cm$^2$ | W % | S % | CTS % |
|---|---|---|---|---|
| Example 1 | 0.10 | 1 | 15 | 10.7 |
| Example 2 | 0.15 | 0.7 | 10.8 | 4.4 |
| Example 3 | 0.22 | 0.4 | 7.9 | 2.8 |
| Comparative Example 1 | 0.49 | 5 | 30 | 35 |
| Comparative Example 2 | 0.36 | 3 | 22 | 22 |
| Comparative Example 3 | 0.25 | 2 | 18 | 15 |
| Comparative Example 4 | 0.05 | 0.31 | 7.5 | 2.5 |
| Comparative Example 5 | 0.04 | 0.23 | 7.3 | 2.2 |
| Comparative Example 6 | 0.35 | 5 | 25 | 20 |
| Comparative Example 7 | 0.26 | 3 | 22 | 18 |
| Comparative Example 8 | 0.06 | 0.3 | 7.7 | 3.7 |

Result analysis: comparing the above data, it can be seen that increasing the amount of the porous carbon material loaded with zinc oxide in the spinning raw material will reduce the haze attenuation rate and the attenuation rate of the heat shielding rate. However, after increasing to a certain amount, the decrease rate will significantly slow down. After increasing the amount of the surfactant in the spinning solvent, the haze attenuation rate and the attenuation rate of the heat shielding rate will also decrease. However, after increasing to a certain amount, the decrease rate will also significantly slow down.

The specific embodiments described herein are merely illustrative of the spirit of the present disclosure. It is apparent to those skilled in the art that various modifications, amendments and alternatives can be made to the described embodiments without departing from the spirit of the present disclosure or scope defined by the appended claims.

**Claims**

1. A flash evaporation material with a low haze attenuation rate, wherein a raw material of the flash evaporation material comprises polyethylene, and a grammage of a flash spun non-woven fabric is greater than 35 $g/m^2$,

   a compression specific power P of the flash evaporation material is greater than 0.07 $gf \cdot cm/cm^2$;
   the compression specific power is tested by a KES style tester, and the compression specific power is tested by FB3;
   a haze attenuation rate $\triangle W$ of the flash evaporation material is 0.3%-1.5%;

$$\triangle W=(W0-W5)/W0*100\%;$$

   an attenuation rate $\triangle S$ of a heat shielding rate of the flash evaporation material is 7%-15%;

$$\triangle S=(S0-S5)/S0*100\%;$$

   tests for a heat shielding rate, a haze and a transverse tensile strength:

   (1) placing a sample at 25°C and a relative humidity of 65% for 24 hours; then measuring and recording a shading index thereof as an initial heat shielding rate S0, measuring and recording a haze thereof as an initial haze W0, and measuring and recording a transverse tensile strength thereof as a transverse tensile strength CTS0;
   (2) then exposing the sample to a dry and hot atmosphere of 90°C for 6 hours, and then cooling at 25°C and a relative humidity of 65% for 24 hours;
   (3) repeating the operation of step (2), and after treatment for 4 times, measuring and recording a shading index thereof as a final heat shielding rate S5, measuring and recording a haze thereof as a final haze W5, and measuring and recording a transverse tensile strength thereof as a final transverse tensile strength CTS5;

   the haze is tested according to the national standard GB/T2410-2008; and
   the heat shielding rate is measured according to the national standard GB/T41560-2022.

2. The flash evaporation material with a low haze attenuation rate of claim 1, wherein the grammage of the flash spun non-woven fabric is less than 75 $g/m^2$.

3. The flash evaporation material with a low haze attenuation rate of claim 1, wherein the compression specific power P of the flash spun non-woven fabric is greater than 0.15 $gf \cdot cm/cm^2$.

4. The flash evaporation material with a low haze attenuation rate of claim 1, wherein the haze attenuation rate $\triangle W$ of the flash evaporation material is 0.6%-0.9%.

5. The flash evaporation material with a low haze attenuation rate of claim 1, wherein the haze attenuation rate $\triangle W$ of the flash evaporation material is 0.9%-1.3%.

6. The flash evaporation material with low haze attenuation rate of claim 1, wherein the heat shielding rate attenuation rate of the flash evaporation material is 7%-10%.

7. The flash evaporation material with a low haze attenuation rate of claim 1, wherein the rate attenuation rate of a heat shielding rate of the flash evaporation material is 10%-14%.

8. The flash evaporation material with a low haze attenuation rate of claim 1, wherein an attenuation rate $\triangle$CTS of a transverse tensile strength of the flash evaporation material is 2%-10%;

$$\triangle CTS=(CTS0-CTS5)/CTS0*100\%;$$

and
the transverse tensile strength CTS0 is greater than 1.4 KN/m.

9. The flash evaporation material with a low haze attenuation rate of claim 8, wherein the attenuation rate $\triangle$CTS of a transverse tensile strength of the flash evaporation material is 3%-5%.

10. The flash evaporation material with a low haze attenuation rate of claim 8, wherein an attenuation rate $\triangle$CTS of a transverse tensile strength of the flash evaporation material is 5%-7%.

11. The flash evaporation material with a low haze attenuation rate of claim 1, wherein a spinning raw material includes polyethylene and a porous carbon material loaded with zinc oxide, and a spinning solvent includes a surfactant and a solvent.

12. The flash evaporation material with a low haze attenuation rate of claim 11, wherein a mass fraction of the porous carbon material loaded with zinc oxide in the spinning raw material is 3%-6%; and
the surfactant in the spinning solvent is 500 ppm-2000 ppm.

13. The flash evaporation material with a low haze attenuation rate of claim 11, wherein the porous carbon material loaded with zinc oxide is prepared by the following steps:

(1) preparation of a solution of silica nanoparticles surface-coated with polyvinyl alcohol:
(2) preparation of a zinc-containing porous carbon precursor dispersion:
further adding the solution of silica nanoparticles surface-coated with polyvinyl alcohol prepared in step (1) to a zinc nitrate aqueous solution to continue a first-order reaction, thus making zinc and polyvinyl alcohol perform a chelation reaction and being loaded on the surface of the silica nanoparticles, then using an alkali to slowly adjust a pH value of a mixed solution to 11-12, and continuing a second-order reaction under alkali conditions to obtain the zinc-containing porous carbon precursor dispersion;
(3) preparation of the porous carbon material loaded with zinc oxide:
finally, separating the zinc-containing porous carbon precursor dispersion in step (2) by centrifugation, and taking a sediment from a lower layer to obtain a zinc-containing porous carbon precursor; performing aerobic calcination on the zinc-containing porous carbon precursor in the lower layer aerobically at 180°C-200°C for 15-30 minutes, and then rapidly heating up to 440°C-450°C within 1-5 minutes, performing anaerobic calcination for 45-60 minutes, and obtaining the porous carbon material loaded with zinc oxide after natural cooling.

## INTERNATIONAL SEARCH REPORT

| | International application No. |
|---|---|
| | **PCT/CN2023/123169** |

**A. CLASSIFICATION OF SUBJECT MATTER**

D04H1/724(2012.01)i

According to International Patent Classification (IPC) or to both national classification and IPC

**B. FIELDS SEARCHED**

Minimum documentation searched (classification system followed by classification symbols)

IPC:D04H

Documentation searched other than minimum documentation to the extent that such documents are included in the fields searched

Electronic data base consulted during the international search (name of data base and, where practicable, search terms used)

CNTXT, ENTXTC, VEN: 布, 多孔碳, 纺丝, 非织造, 减少, 减小, 聚乙烯, 片材, 闪纺, 闪蒸, 衰减, 缩减, 透明, 透明度, 透明度减少, 透明度减小, 透明度衰减, 透明度损失, 无纺布, 雾度, 雾度减少, 雾度减小, 雾度衰减, 雾度损失, 氧化锌, 遮热, 遮阳, 织物, haze, nonwoven, PE, silica, zinc, ZNO, heat shield, flash, spin, spun, non-woven, porous, carbon.

**C. DOCUMENTS CONSIDERED TO BE RELEVANT**

| Category* | Citation of document, with indication, where appropriate, of the relevant passages | Relevant to claim No. |
|---|---|---|
| PX | CN 116623367 A (JIANGSU QINGYUN NEW MATERIAL CO., LTD.) 22 August 2023 (2023-08-22) <br> claims 1-13 | 1-13 |
| A | CN 116356482 A (JIANGSU QINGYUN NEW MATERIAL CO., LTD.) 30 June 2023 (2023-06-30) <br> description, paragraphs 4-27 | 1-13 |
| A | CN 104282913 A (ANHUI NORMAL UNIVERSITY) 14 January 2015 (2015-01-14) <br> entire document | 1-13 |
| A | CN 114687057 A (ZHEJIANG QINGYUN NEW MATERIAL TECHNOLOGY CO., LTD.) 01 July 2022 (2022-07-01) <br> entire document | 1-13 |
| A | CN 115198445 A (JIANGSU QINGYUN NEW MATERIAL TECHNOLOGY CO., LTD.) 18 October 2022 (2022-10-18) <br> entire document | 1-13 |

☑ Further documents are listed in the continuation of Box C. ☑ See patent family annex.

| | |
|---|---|
| \* Special categories of cited documents: <br> "A" document defining the general state of the art which is not considered to be of particular relevance <br> "D" document cited by the applicant in the international application <br> "E" earlier application or patent but published on or after the international filing date <br> "L" document which may throw doubts on priority claim(s) or which is cited to establish the publication date of another citation or other special reason (as specified) <br> "O" document referring to an oral disclosure, use, exhibition or other means <br> "P" document published prior to the international filing date but later than the priority date claimed | "T" later document published after the international filing date or priority date and not in conflict with the application but cited to understand the principle or theory underlying the invention <br> "X" document of particular relevance; the claimed invention cannot be considered novel or cannot be considered to involve an inventive step when the document is taken alone <br> "Y" document of particular relevance; the claimed invention cannot be considered to involve an inventive step when the document is combined with one or more other such documents, such combination being obvious to a person skilled in the art <br> "&" document member of the same patent family |

| Date of the actual completion of the international search | Date of mailing of the international search report |
|---|---|
| **12 March 2024** | **29 March 2024** |

| Name and mailing address of the ISA/CN | Authorized officer |
|---|---|
| **China National Intellectual Property Administration (ISA/CN)** <br> **China No. 6, Xitucheng Road, Jimenqiao, Haidian District, Beijing 100088** | |
| | Telephone No. |

Form PCT/ISA/210 (second sheet) (July 2022)

## INTERNATIONAL SEARCH REPORT

International application No.

**PCT/CN2023/123169**

**C.    DOCUMENTS CONSIDERED TO BE RELEVANT**

| Category* | Citation of document, with indication, where appropriate, of the relevant passages | Relevant to claim No. |
|---|---|---|
| A | US 5147586 A (E. I. DU PONT DE NEMOURS AND COMPANY) 15 September 1992 (1992-09-15) <br> entire document | 1-13 |
| A | US 6010970 A (E. I. DU PONT DE NEMOURS AND COMPANY) 04 January 2000 (2000-01-04) <br> entire document | 1-13 |

Form PCT/ISA/210 (second sheet) (July 2022)

EP 4 745 284 A1

## INTERNATIONAL SEARCH REPORT
### Information on patent family members

International application No.

**PCT/CN2023/123169**

| Patent document cited in search report | | | Publication date (day/month/year) | Patent family member(s) | | | Publication date (day/month/year) |
|---|---|---|---|---|---|---|---|
| CN | 116623367 | A | 22 August 2023 | CN | 116623367 | B | 22 September 2023 |
| CN | 116356482 | A | 30 June 2023 | None | | | |
| CN | 104282913 | A | 14 January 2015 | CN | 104282913 | B | 18 January 2017 |
| CN | 114687057 | A | 01 July 2022 | CN | 114687057 | B | 09 June 2023 |
| CN | 115198445 | A | 18 October 2022 | None | | | |
| US | 5147586 | A | 15 September 1992 | DE | 69202455 | D1 | 14 June 1995 |
| | | | | DE | 69202455 | T2 | 15 February 1996 |
| | | | | ES | 2072758 | T3 | 16 July 1995 |
| | | | | EP | 0572570 | A1 | 08 December 1993 |
| | | | | EP | 0572570 | B1 | 10 May 1995 |
| | | | | AU | 1585592 | A | 15 September 1992 |
| | | | | JPH | 06505536 | A | 23 June 1994 |
| | | | | JP | 3034042 | B2 | 17 April 2000 |
| | | | | KR | 930703486 | A | 30 November 1993 |
| | | | | KR | 0178284 | B1 | 01 February 1999 |
| | | | | WO | 9214870 | A1 | 03 September 1992 |
| | | | | MX | 9200729 | A | 01 October 1992 |
| | | | | CA | 2103921 | A1 | 23 August 1992 |
| | | | | CA | 2103921 | C | 21 May 2002 |
| | | | | US | 6291566 | B1 | 18 September 2001 |
| US | 6010970 | A | 04 January 2000 | BR | 9807721 | A | 02 May 2000 |
| | | | | JP | 2001515544 | A | 18 September 2001 |
| | | | | KR | 20000075962 | A | 26 December 2000 |
| | | | | CA | 2279865 | A1 | 11 September 1998 |
| | | | | DE | 69802670 | D1 | 10 January 2002 |
| | | | | DE | 69802670 | T2 | 01 August 2002 |
| | | | | EP | 0964949 | A1 | 22 December 1999 |
| | | | | EP | 0964949 | B1 | 28 November 2001 |
| | | | | ES | 2165671 | T3 | 16 March 2002 |
| | | | | WO | 9839509 | A1 | 11 September 1998 |
| | | | | CN | 1249791 | A | 05 April 2000 |

Form PCT/ISA/210 (patent family annex) (July 2022)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- CN 114687057 A
- CN 114687069 A
- CN 114687068 A
- CN 114108112 A
- CN 115537959 A
- CN 109154138 A
- US 20160138197 A1
- US 8048513 B